# EUROPÄISCHE PATENTSCHRIFT

(11) **EP 0 567 622 B1**
(45) Veröffentlichungstag und Bekanntmachung des Hinweises auf die Patenterteilung: **24.04.1996**
(21) Anmeldenummer: 92923736.0
(22) Anmeldetag: 17.11.1992
(51) Int. Cl.: C09B 11/24, C09B 11/28, G01N 33/533, G01N 33/58

(54) **NEUE PENTACYCLISCHE VERBINDUNGEN UND IHRE VERWENDUNG ALS ABSORPTIONS- ODER FLUORESZENZFARBSTOFFE**
NEW PENTACYCLIC COMPOUNDS AND THEIR USE AS ABSORPTION OR FLUORESCENCE DYES
NOUVEAUX COMPOSES PENTACYCLIQUES ET LEUR UTILISATION COMME COLORANTS PAR ABSORPTION OU PAR FLUORESCENCE

(30) Priorität: 18.11.1991 DE 4137934
(43) Veröffentlichungstag der Anmeldung: 03.11.1993
(73) Patentinhaber: BOEHRINGER MANNHEIM GMBH, D-68298 Mannheim (DE)
(72) Erfinder: HERRMANN, Rupert, D-8120 Weilheim (DE); JOSEL, Hans-Peter, D-8120 Weilheim (DE); DREXHAGE, Karl-Heinz, D-5900 Siegen (DE); ARDEN-JACOB, Jutta, D-5600 Siegen (DE)
(74) Vertreter: Huber, Bernhard, Dipl.-Chem.
(86) Internationale Anmeldenummer: EP9202645
(87) Internationale Veröffentlichungsnummer: WO9310189

(56) Entgegenhaltungen:
- EP-A- 0 285 179
- EP-A- 0 357 350
- DE-A- 3 322 945
- CHEMICAL ABSTRACTS, vol. 109, no. 18, 1988, Columbus, Ohio, US; abstract no.
- 151427, Seite 102 ;Spalte 2 ;

## Beschreibung

Die Erfindung betrifft neue pentacyclische Verbindungen mit einer Rhodamin-ähnlichen Struktur, sowie deren Verwendung als Absorptions- und insbesondere Fluoreszenzfarbstoffe.

Es sind zahlreiche Rhodamin-Derivate bekannt, die als hydrophile Derivate mit entsprechenden reaktiven Gruppen als Marker (Label-Substanz) eingesetzt werden können. Nach Umsetzung von z.B. N-Hydroxysuccinimidestern von Tetramethylcarboxyrhodamin oder Carboxyrhodamin 101 (RHODOS) mit aminogruppenhaltigen Proteinen oder Haptenen erhält man z.B. entsprechend markierte Verbindungen, die in diagnostischen Systemen eingesetzt werden können.

Beispiele für derartige Hapten-Fluoreszenz-Konjugate (HFK) sind in der EP-A-285179 beschrieben; die dort beschriebenen Verbindungen besitzen jedoch Absorptionsmaxima bei Wellenlängen < 600 nm.

Es sind nur wenige Rhodamine mit Absorptionsmaxima > 600 nm bekannt, z.B. Rhodamin 700/800. Die Herstellung von Konjugaten dieser Verbindungen ist nicht beschrieben.

Für diagnostische Systeme ist es in der Regel zweckmäßig, preisgünstige Lichtquellen einsetzen zu können, wie z.B. Laserdioden (670/760 nm) oder billige Laser (He/Ne:633 nm); diese Lichtquellen können jedoch für die bisher bekannten, und z.B. für Hapten-Fluoreszenz-Konjugate eingesetzten Rhodamin-Verbindungen mit einen Absorptionsmaximum < 600 nm nicht verwendet werden.

Für diagnostische Anwendungen werden ebenfalls, je nach Analyt-Konzentrationen, Kombinationen aus Fluoreszenz-Latices mit Hapten-Fluoreszenz-Konjugaten eingesetzt. Auch solche Kombinationen sind für die vorstehend genannten günstigen Wellenlängenbereiche, bei denen preisgünstige Lichtquellen verwendet werden können, bisher nicht beschrieben.

Aufgabenstellung der vorliegenden Erfindung ist deshalb die Bereitstellung von neuen pentacyclischen Verbindungen mit einer Rhodamin-artigen Struktur, deren Absorptionsmaxima in einem Wellenlängenbereich > 600 nm, vorzugsweise > 630 nm, liegen, und mit denen Hapten-Fluoreszenz-Konjugate oder/und Fluoreszenz-Latices hergestellt werden können, für die auch der Einsatz preisgünstiger Lichtquellen, wie der vorstehend genannten Laserdioden oder billigen Laser, möglich ist. Diese Aufgabenstellung wird mit der vorliegenden Erfindung gelöst.

Gegenstand der Erfindung sind pentacyclische Derivate der allgemeinen Formel Ia, Ib und Ic 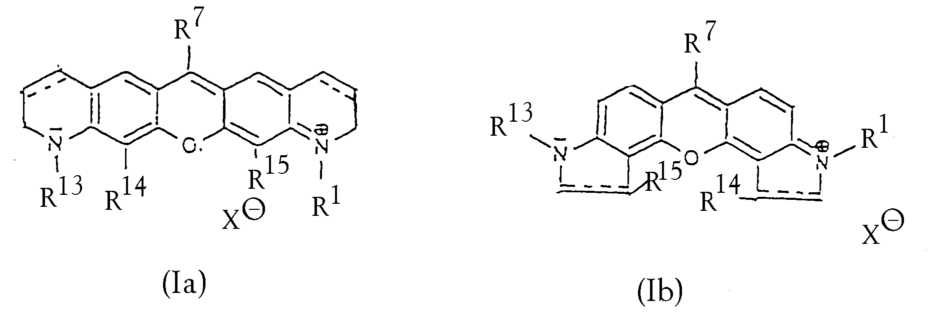 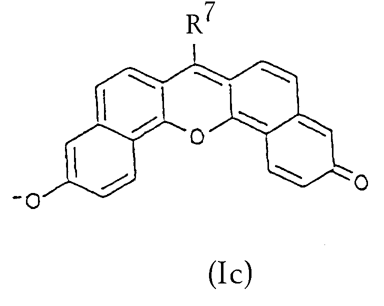 worin R¹ und R¹³ gleich oder verschieden sind und bedeuten:
Wasserstoff, Alkyl mit 1 bis 20, vorzugsweise 1 bis 7 Kohlenstoffatomen, Polyoxyhydrocarbyl-Einheiten, Phenyl, Phenylalkyl mit 1 bis 3 Kohlenstoffatomen in der Alkylkette, worin die Alkyl- oder/und Phenylreste durch eine oder mehrere Hydroxy-, Halogen-, Sulfo-, Carboxy- und Alkoxycarbonylgruppen, worin Alkoxy 1 bis 4 Kohlenstoffatome besitzen kann, substituiert sein können;
R⁷ eine durch mindestens ein Halogen substituierte Alkylgruppe mit 1 bis 20, vorzugsweise 1 bis 7 Kohlenstoffatomen, oder eine Phenylgruppe, die durch eine in o-Stellung zu dem an das pentacyclische Ringsystem gebundene Kohlenstoffatom befindliche Carboxy- oder Alkoxycarbonylgruppe und mindestens ein Halogen substituiert ist, und worin Alkoxy 1 bis 4 Kohlenstoffatome besitzen kann, oder eine Carboxyalkylgruppe oder eine Carboxymethylen-oxy-alkyloxygruppe bedeutet;
die beiden strichliniert eingezeichneten Bindungen der allgemeinen Formel (Ia) und (Ib) bedeuten, daß die beiden damit verbundenen Kohlenstoffatome durch eine Einfach- oder Doppelbindung miteinander verbunden sein können, wobei Doppelbindungen bevorzugt sind,
wobei R¹⁴, R¹⁵ und die übrigen, nicht mit spezifischen Symbolen bezeichneten Positionen des pentacyclischen Grundkörpers durch Alkylgruppen mit 1 bis 20 Kohlenstoffatomen substituiert sein können;
wobei R¹ bzw. R¹³ bei Verbindungen der Formel Ia nicht mit Substituenten an den Positionen R¹⁵ bzw. R¹⁴ verbrückt sind, und X⁻ ein Gegenion ist; sowie deren zumindest an einem der Reste R¹, R⁷ oder/und R¹³ aktivierten Derivate.

Bevorzugte Ausführungsformen der erfindungsgemäßen Verbindungen der Formel (Ia) und (Ib) sind Gegenstand der Ansprüche 2 bis 9. Konjugate, die eine erfindungsgemäße Verbindung enthalten, sind Gegenstand von Anspruch 10.

Unter dem Begriff "Polyoxyhydrocarbyl-Einheiten" im Sinne der vorliegenden Erfindung sind polymere oder oligomere organische Reste zu verstehen, die über O-Brücken miteinander verknüpft sind. Insbesondere sind unter diesem Begriff Polyether, Polyole, lösliche Carbohydrate und Derivate davon oder wasserlösliche Polymere zu verstehen. Besonders bevorzugt sind Polyethylenoxygruppen, deren Größe so ist, daß das Molekulargewicht der Gesamtverbindung 800 bis 1200, vorzugsweise etwa 1000 ist. Die obengenannten Polyethylenoxygruppen in der Bedeutung von R¹ oder/und R¹³ bewirken eine Verbesserung der Löslichkeit, vermindern die unspezifische Bindung der Verbindung an Proteine und verhindern eine Dimerisierung.

Unter dem Begriff "Carboxyalkylgruppe" für R⁷ ist insbesondere eine Carboxyalkylgruppe mit einer Alkylenkettenlänge von 0-10 zu verstehen, wobei R⁷ in diesem Fall bei einer Alkylkettenlänge von 0 eine Carbonsäuregruppe bedeutet. Bei einer Carboxymethylenmethylen-oxy-alkyloxygruppe bedeutet Alkyl vorzugsweise 1-10 Kohlenstoffatome, besonders bevorzugt 1 bis 4 Kohlenstoffatome.

Bevorzugt sind auch Verbindungen, welche dadurch gekennzeichnet sind, daß eine oder beide der strichlinierten Bindungen der Formeln Ia oder/und Ib Doppelbindungen sind.

Diese Einführung von einer oder zwei Doppelbindungen in den strichlinierten Positionen der Formel (Ia) führt überraschenderweise zu einer besonders langwelligen Verschiebung.

Die in den Formeln mit R¹⁴ und R¹⁵ sowie die nicht mit spezifischen Symbolen bezeichneten Positionen der Ringe können durch gegebenenfalls substituiertes Alkyl mit 1-20, insbesondere 1-7 Kohlenstoffatomen substituiert sein.

Bei Verbindungen der Formel (Ia) sind die Substituenten R¹ bzw. R¹³ nicht mit Substituenten an den Positionen R¹⁵ bzw. R¹⁴ verbrückt. Dies hat insbesondere, wenn R⁷ ein halogensubstituierter Alkylrest ist, eine überraschende Erhöhung der Quantenausbeute zur Folge.

Eine Alkylgruppe mit 1 bis 7 Kohlenstoffatomen kann verzweigtkettig oder geradkettig sein; sie besitzt vorzugsweise 1 bis 4 Kohlenstoffatome, und ist insbesondere z.B. Methyl, Ethyl, Propyl, Isopropyl, Butyl, iso-Butyl oder tert.-Butyl.

Eine Alkoxygruppe in einer Alkoxycarbonylgruppe von R⁷ besitzt vorzugsweise 1 oder 2 Kohlenstoffatome.

Wenn die Alkylgruppe für die Bedeutung R⁷ steht, besitzt sie insbesondere 1 bis 3 Kohlenstoffatome, und für die übrigen Reste außer R¹ und R¹³ vorzugsweise 1 oder 2 Kohlenstoffatome.

Eine Phenylalkylgruppe mit 1 bis 3 Kohlenstoffatomen ist insbesondere eine Phenethyl- oder Benzylgruppe.

In den erfindungsgemäßen Verbindungen der Formel (Ia) und (Ib) liegt vorzugsweise zumindest einer der Reste R¹, R⁷ oder/und R¹³, und insbesondere der Rest R⁷, in Form eines aktivierten Derivates vor. Ein solches aktiviertes Derivat leitet sich insbesondere von einer Carbonsäure- oder Sulfonsäuregruppierung ab, und ist z.B. ein Säureester, ein Säurehalogenid, vorzugsweise Bromid, und insbesondere Chlorid, oder ein N-Hydroxy-Succinimidester.

R¹ oder/und R¹³ sind vorzugsweise z.B. Wasserstoff, Alkyl mit 1 bis 4 Kohlenstoffatomen, das gegebenenfalls durch Hydroxy, Halogen, Carboxy, Alkoxycarbonyl oder Sulfo substituiert ist, oder Polyoxyhydrocarbyl-Einheiten, vorzugsweise Polyether, Polyole, lösliche Carbohydrate und Derivate davon (z.B. durch Amino- oder Säurefunktionen substituierte Carbohydrate), oder wasserlösliche Polymere oder Benzyl. R⁷ kann vorzugsweise eine perhalogenierte Alkylgruppe mit 1 bis 3 Kohlenstoffatomen oder ein o-Carboxy-oder o-Alkoxycarbonyltetrahalophenylgruppe sein, worin Alkoxy die vorstehend angegebene Bedeutung besitzt; in einer perhalogenierten Alkyl- oder tetrahalogenierten o-Carboxy- oder o-Alkoxycarbonyl-phenylgruppe bedeutet Halogen Iod, Brom, insbesondere Chlor und in erster Linie Fluor. Eine perfluorierte Alkylgruppe ist z.B. Trifluormethyl oder Heptafluorpropyl. Bei einer ebenfalls bevorzugten Carboxyphenylgruppe in der Bedeutung von R⁷ befindet sich die Carboxygruppe in o-Stellung zu dem an das pentacyclische Ringsystem gebundenen Kohlenstoffatom. Die Einführung von mindestens einem oder mehreren Halogenatomen führt dabei überraschenderweise zu einer langwelligen Verschiebung gegenüber bekannten nicht-halogensubstituierten Verbindungen (EP-A- 0 285 179).

Als Gegenion läßt sich jedes zur Ladungsneutralisierung geeignete und mit dem kationischen Grundgerüst kompatible Anion verwenden; bevorzugt wird Perchlorat eingesetzt, oder aber das Gegenion von einer Carboxy- oder Sulfo-Gruppe einer der Reste abgeleitet. Durch Wahl eines geeigneten Gegenions läßt sich zusätzlich zur Auswahl und Kombination der Reste der je nach dem beabsichtigten Anwendungszweck gewünschte Grad der Lipophile optimieren. Neben einem Einbau längerer lipophiler Reste, z.B. des Heptafluorpropylrestes anstelle des Trifluormethylrestes in der Bedeutung von R⁷, oder/und von längeren und verzweigten Alkylresten, oder/und von Arylresten, insbesondere in der Bedeutung der Reste R¹ und R¹³, läßt sich z.B. eine weitere Steigerung der Lipophilie durch einen gezielten Anionenaustausch ermöglichen, z.B. durch den Ersatz von Perchlorat durch Heptafluorbutyrat.

Beispiele für besonders bevorzugte Substituenten in der Bedeutung von R¹ und R¹³ sind: Wasserstoff, Methyl, Ethyl, 3-Sulfopropyl, 4-Sulfobutyl, 3-Carboxypropyl, 4-Carboxybutyl, 3-Methoxycarbonylpropyl, 3-Ethoxycarbonylpropyl, Methoxyethoxy-ethyl, Hydroxy-ethoxy-ethyl, Benzyl. Zur Verwendung als hydrophile Marker kann es zweckmäßig sein, unsymmetrisch substituierte Produkte einzusetzen, in denen die Reste R¹ und R¹³ verschieden sind, und z.B. eine 3-Carboxypropyl oder 4-Carboxybutylgruppe (R¹) und eine 3-Sulfo-Propyl- oder 4-Sulfo-Butyl-Gruppe (R¹³) bedeuten.

Bevorzugte Beispiele für den Rest R⁷ sind: Trifluormethyl, Pentafluorethyl, Heptafluorpropyl, 3,4,5,6-Halo-2-carboxy- oder -ethoxycarbonyl-phenyl (Halo = Brom, Iod und insbesondere Chlor oder Fluor), oder Carboxyethyl.

Besonders bevorzugte erfindungsgemäße Verbindungen der Formel (Ia), (Ib) oder (Ic) sind die nachfolgend angegebenen Verbindungen (1) bis (24); für die Absorptionsmaxima (λ_{A}), die Fluoreszenzmaxima (λ_{F} ) und die Fluoreszenzquantenausbeuten (Q) angegeben. Die Angaben beziehen sich, wenn nicht anders angegeben, auf leicht angesäuertes Ethanol als Lösungsmittel bei 20°C.

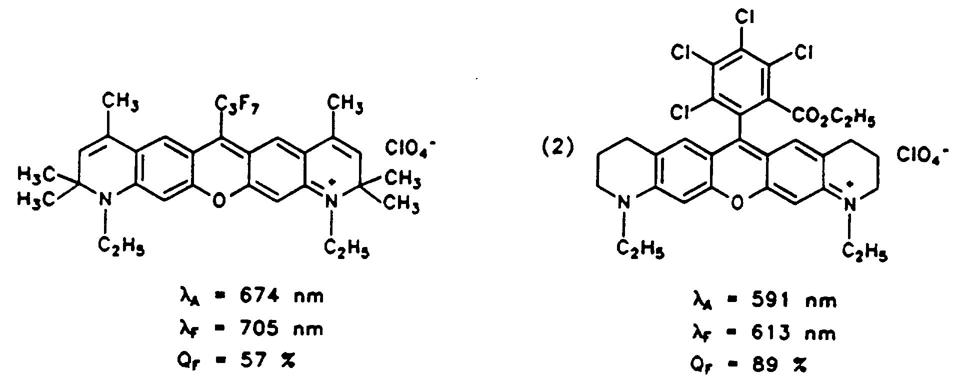

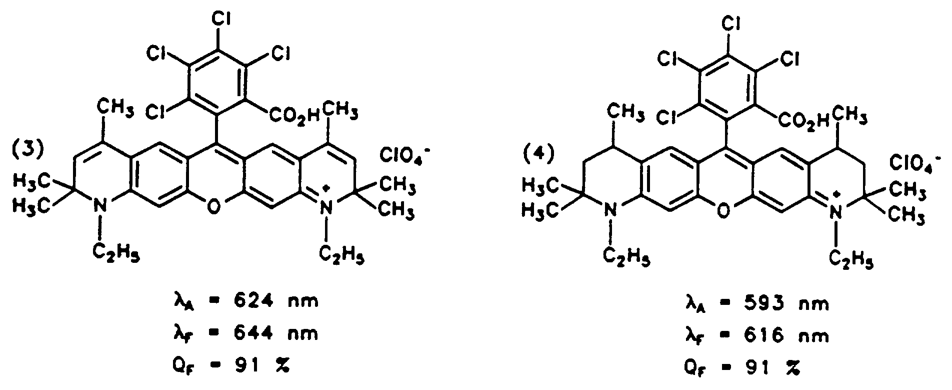

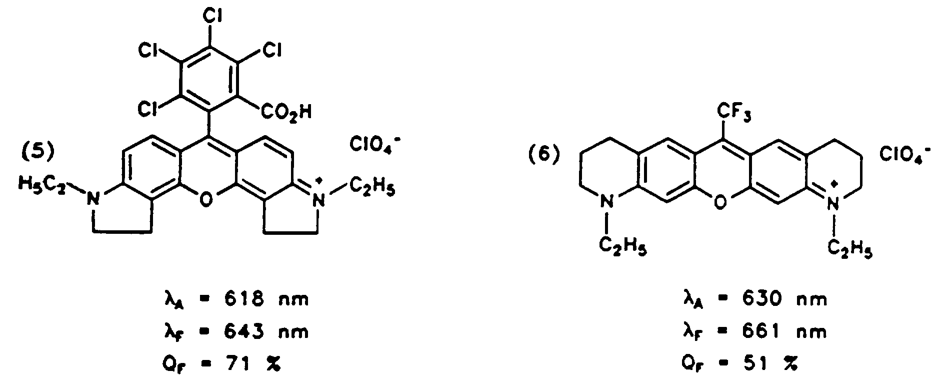

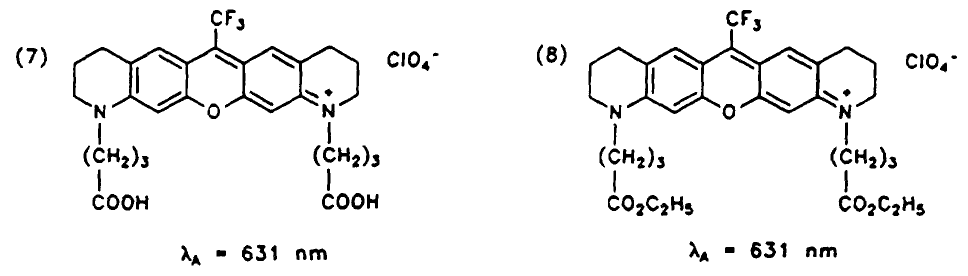

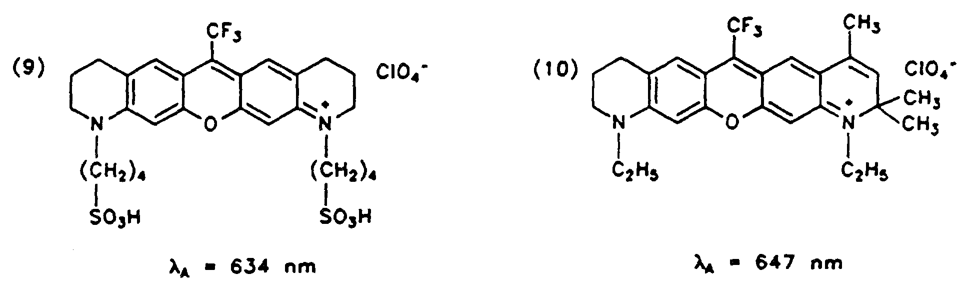

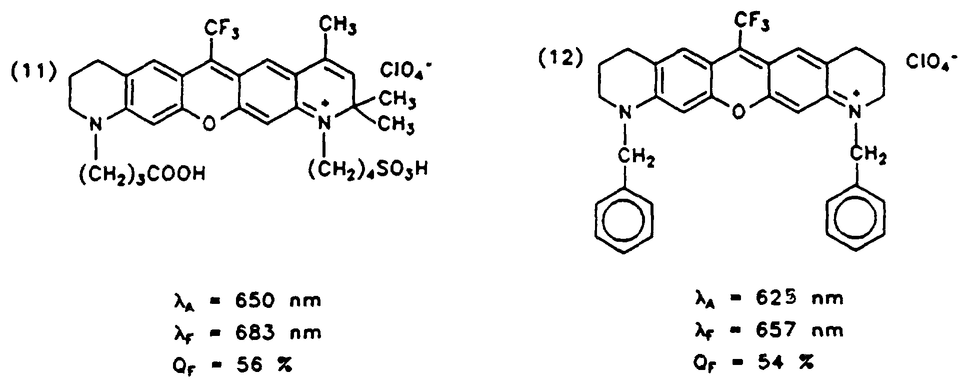

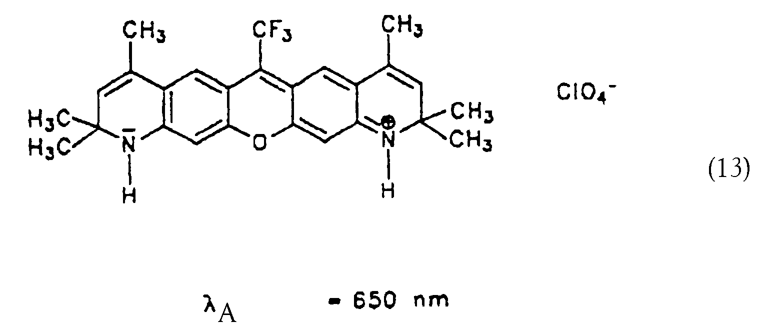

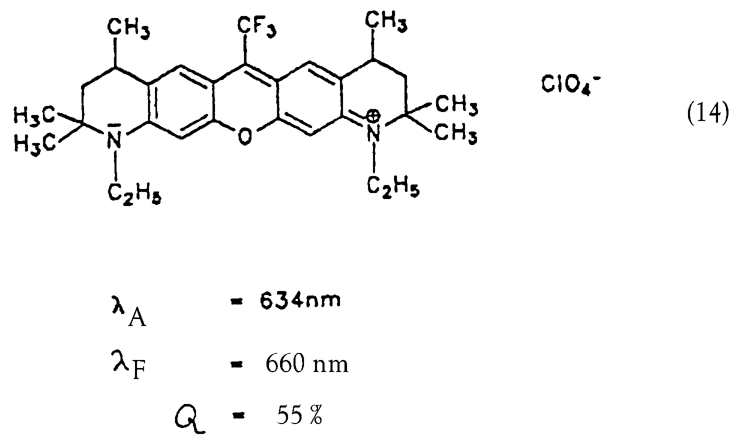

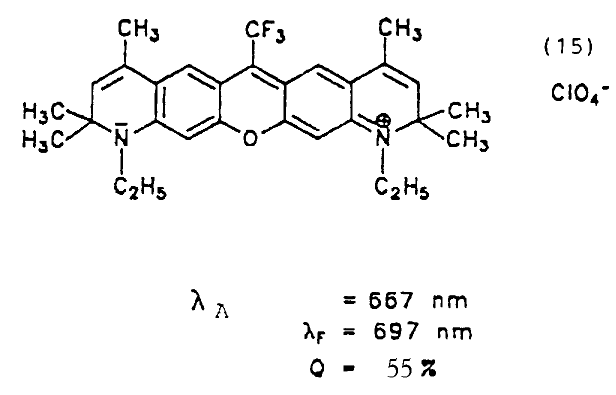

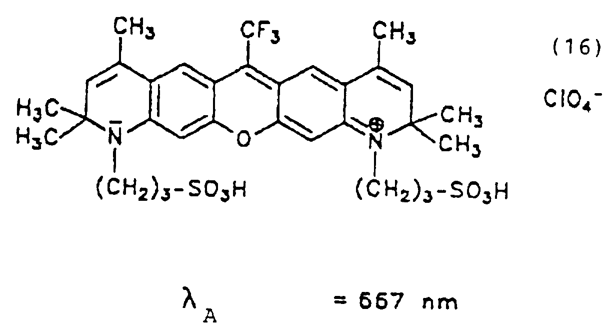

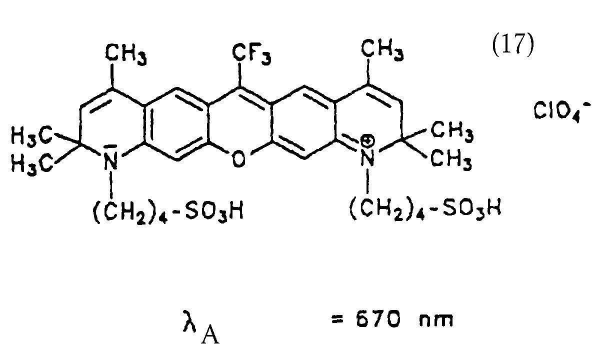

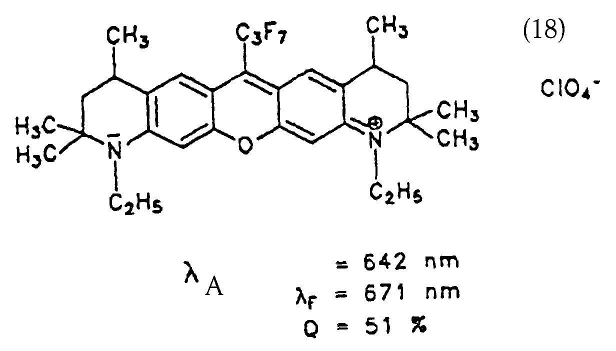

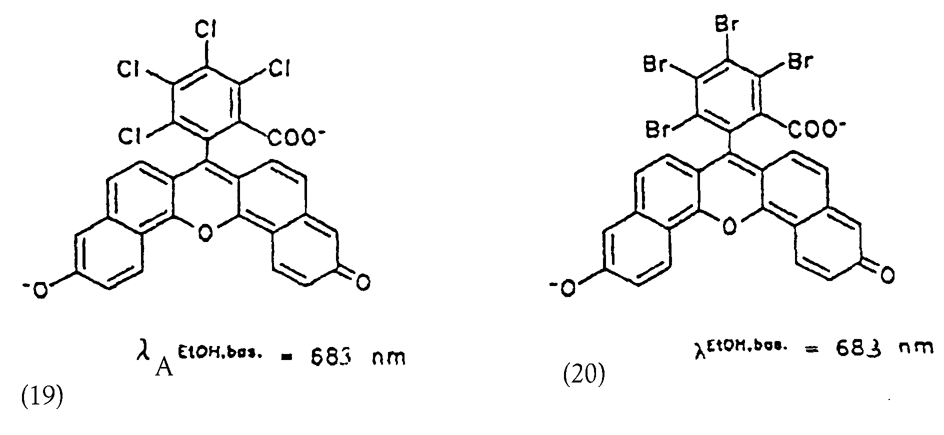

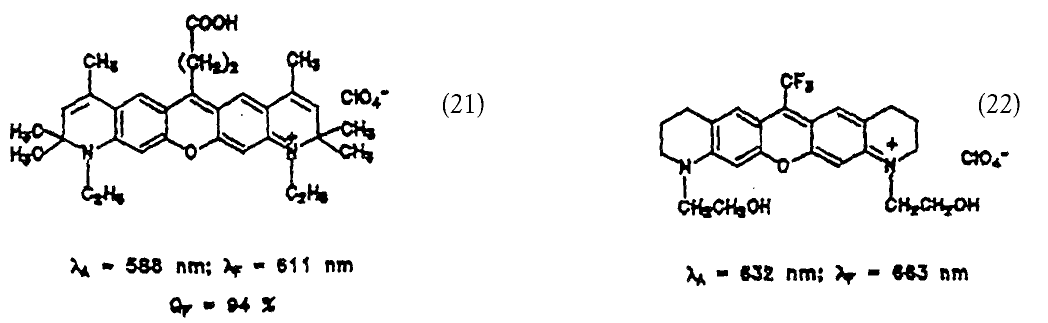

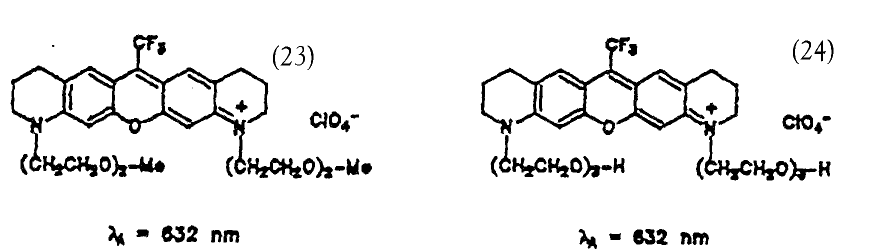

Für die erfindungsgemäßen Verbindungen (Ia) und (Ib), in denen R⁷ halogeniertes o-Carboxy- oder Alkoxycarbonyl-phenyl bedeutet, kann für die Herstellung der erfindungsgemäßen neuen Verbindungen auf die für die Synthese von Rhodaminen übliche Art und Weise durch Kondensation von 2 Mol eines entsprechenden 3-Aminophenolderivats (z.B. von N-Ethyl-7-hydroxy-2,2,4-trimethyl-1,2,3,4-tetrahydrochinolin) mit 1 Mol eines entsprechenden Anhydrids (z.B. Phthalsäureanhydridderivat erfolgen (vgl. z.B. Römpps' Chemie Lexikon, 8. Auflage, Seite 3580). Die Ausgangsverbindungen sind bekannt oder lassen sich auf an sich bekannte Weise erhalten.

Erfindungsgemäße Verbindungen, in denen R⁷ eine durch Halogen substituierte Alkylgruppe, wie z.B. CF₃, darstellt, lassen sich z.B. nach dem für Rhodamin 700 angegebenen Verfahren (vgl. N.F. Haley, J. Heterocyclic Chemistry, 14, 683 (1977) herstellen.

Überraschenderweise gelingt die Synthese der erfindungsgemäßen Verbindungen, in denen R⁷ ein Halogenalkyl bedeutet, in guten Ausbeuten durch Umsetzung der entsprechenden Aminophenyl-alkylether mit den jeweiligen Halogensäureanhydriden gemäß dem nachfolgenden allgemeinen Reaktionsschema:

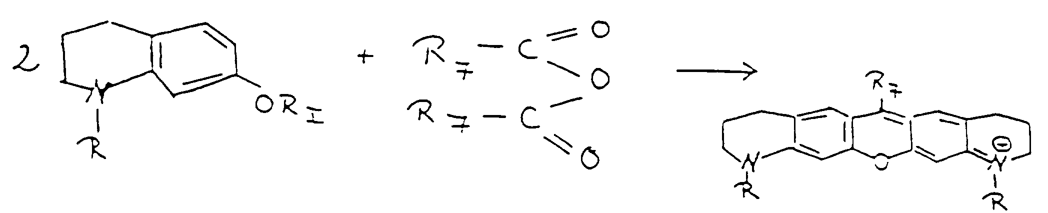

Danach wird der entsprechende Aminophenyl-alkylether bei Raumtemperatur unter Inertbedingungen, z.B. unter Stickstoffatmosphäre, mit dem Halogensäureanhydrid inkubiert und anschließend bei erhöhter Temperatur (vorzugsweise ca. 100°C) mit konzentrierter Säure (vorzugsweise mit ca. 70 %-ige Schwefelsäure) nachbehandelt.

Die Verbindungen der Formel (Ic) lassen sich erhalten durch Umsetzung der entsprechenden Diphenole mit den jeweiligen Anhydriden. Das aus dem Stand der Technik bekannte Rhodamin 700 (vgl. T.F. Johnston, R.H. Brady, W. Proffitt, Appl. Optics. 21(13), 2307 (1982)), besitzt ein Absorptionsmaximum von 643 nm bei einer Quantenausbeute von ca. 20 %; diese Verbindung unterscheidet sich von den erfindungsgemäßen Verbindungen dadurch, daß die Reste R¹³ und R¹⁴, sowie R¹ und R¹⁵ in Formel (Ia) überbrückt sind. Bei den neuen erfindungsgemäßen Verbindungen der Formel (Ia), bei denen diese Überbrückung fehlt, wird zwar die Wellenlänge nicht in den langwelligeren Bereich verschoben, es steigt aber überraschenderweise die Quantenausbeute auf Werte zwischen 50 % (vgl. z.B. die Verbindung 18) und 70 % (vgl. Verbindung 6) an. Durch die Einführung einer oder zwei Doppelbindungen erfolgt eine Verschiebung in den langwelligen Bereich; dabei wird die Quantenausbeute überraschenderweise nicht wesentlich verringert, wie dies üblicherweise durch Vergrößerung des II-Systems zu erwarten ist.

Mit den erfindungsgemäßen Verbindungen werden neue Verbindungen bereitgestellt, die sich aufgrund ihrer spektroskopischen Eigenschaften (Absorptionsmaxima im Bereich von 600 nm und darüber und ein Fluoreszenz zwischen 600 und 700 nm; gute Quantenausbeute) sehr gut für Absorptionsfarbstoffe und insbesondere Fluoreszenzfarbstoffe für die Anwendung in Hapten/- und Antikörper/Protein-Konjugaten und zur Anfärbung von Latices (Fluoreszenz-Latices) eignen. Aufgrund ihrer Eigenschaften eignen sich die erfindungsgemäßen Verbindungen zum Einsatz als Laserfarbstoffe aber auch sehr gut als Absorptionsmedium für Sonnenkollektoren.

Der Substituent R⁷ weist Elektronen-anziehende Substituenten auf, wie z.B. halogenierte Carboxyphenyl-Reste und perfluorierte Alkylreste, mit denen überraschenderweise Farbstoffe mit Absorptionswellenlängen im Bereich von 600 nm oder zum Teil wesentlich darüber erhalten werden können; die zusätzliche Einführung von Doppelbindungen zeigt den gleichen Einfluß und kann ihn noch verstärken.

Durch die Halogenierung des Carboxyphenyl-Restes R⁷ erfolgt z.B. sowohl eine langwellige Verschiebung um ca. 30 nm (von 558 nm bis mindestens 591 nm), sowie eine Erhöhung der Quantenausbeute um ca. 10 %; die Einführung einer oder zwei Doppelbindungen führt dann zu einer weiteren Verschiebung in den längerwelligen Bereich.

Durch entsprechende Derivatisierungen können die erfindungsgemäßen Verbindungen außerdem in aktivierter, kupplungsfähiger Form erhalten werden.

Erfindungsgemäß ist es somit möglich, durch Wahl und Kombination geeigneter Substituenten im Rahmen des erfindungsgemäßen Umfangs Absorptionsfarbstoffe und Fluoreszenzfarbstoffe bereitzustellen, in denen je nach Anwendungszweck die hydrophilen oder lipophilen Eigenschaften überwiegen, und die in breiten Wellenlängenbereichen oberhalb 600 nm einsetzbar sind. Darüberhinaus zeigen die neuen erfindungsgemäßen Verbindungen auch eine stark verbesserte Quantenausbeute, was eine deutliche Empfindlichkeitssteigerung mit sich bringt.

Für die Anwendung in Hapten/Antikörper/Protein-Konjugaten ist es vorteilhaft, wenn die Farbstoffe gut wasserlöslich sind. Für diesen Verwendungszweck werden deshalb vorzugsweise Verbindungen der allgemeinen Formel (Ia) oder (Ib) eingesetzt, in denen R¹ und R¹³ möglichst hydrophil sind. Vorzugsweise sind diese Verbindungen unsymmetrisch substituierte Produkte, die z.B. sowohl Carboxyl- als auch Sulfonsäuregruppen enthalten, und in denen die Reste R¹ und R¹³ verschieden sind. Die Kupplung an das Konjugat erfolgt vorzugsweise über die Reste R¹, R⁷ oder/und R¹³ und insbesondere über eine N-Hydroxysuccinimidgruppierung.

Konjugate der Fluoreszenzfarbstoffe mit Haptenen, wie z.B. Theophylin, Digoxin, T3, T4, oder Proteinen, wie z.B. Antikörper, eignen sich z.B. zum Einsatz in diagnostischen Systemen, insbesondere für Fluoreszenz-Immunoassays.

Eine weitere Anwendungsform für die neuen Verbindungen ist ihre Verwendung zur Anfärbung von Latices. Die Latices werden vorzugsweise dadurch gefärbt, daß geeignete Farbstoffe in diese Partikel adsorptiv integriert werden, wozu es erforderlich ist, daß die als Farbstoffe fungierenden Verbindungen genügend lipophil sind. Eine ausreichende Lipophilie wird dabei insbesondere durch die Auswahl eines geeigneten Substituenten R⁷ erreicht, der möglichst lipophil sein soll. So kann z.B. der lipophile Charakter der Verbindungen der Formel (Ia) oder (Ib) durch Einbau längerer lipophiler Reste in der Bedeutung R⁷ verstärkt werden, z.B. durch Ersatz von Trifluormethyl durch den längeren Heptafluorpropylrest, oder durch Verwendung von längeren und verzweigten Alkylresten, sowie von Arylsubstituenten. Insbesondere eignen sich auch gut Verbindungen der Formel (Ia) und (Ib) mit Rhodaminstruktur, die als Rest R⁷ einen 3,4,5,6-Tetrahalo-2-carboxyphenyl Substituenten enthalten, wobei Halo vorzugsweise Chlor ist. Entsprechend gefärbte Latexpartikel können mit üblichen Methoden mit Proteinen beladen werden und in diagnostischen Systemen eingesetzt werden.

Typische Beispiele für wasserlösliche erfindungsgemäße Farbstoffe für den Einsatz in Hapten/Antikörper/Protein-Konjugaten sind z.B. die vorstehend genannten Verbindungen (7), (9), und insbesondere (11). Beispiele für erfindungsgemäße Verbindungen, die sich sehr gut zum Einbau in Latices eignen, sind z.B. die vorstehend genannten Verbindungen (1), (3), (4), (5), (12) und (18).

Durch eine geeignete Auswahl und Kombinationen der Substituenten läßt sich auch eine Optimierung und Feinabstimmung der Anregungswellenlänge an die zur Verwendung vorgesehenen Lichtquellen vornehmen.

Gegenstand der Erfindung ist deshalb auch die Verwendung der erfindungsgemäßen Verbindungen als Absorptions- oder fluoreszenzfarbstoff, als Anregungs- und Emissionsmedium in einem Farbstofflaser, und insbesondere ihre Verwendung in diagnostischen Systemen; und ihre Verwendung für Hapten/Antikörper/Protein-Konjugate, insbesondere in diagnostischen Systemen.

Für die Verwendung als Marker für Hapten/Antikörper/Protein-Konjugate, insbesondere in diagnostischen Systemen, wird vorzugsweise eine Verbindung der Formel (Ia) und/oder (Ib) verwendet, in der die Reste R¹ und R¹³ so ausgewählt sind, daß sie möglichst hydrophil sind; die Kupplung an das Konjugat erfolgt vorzugsweise über die Reste R¹, R⁷ und/oder R¹³, und in erster Linie mittels einer N-Hydroxysuccinimidgruppierung.

Eine weitere Verwendung der erfindungsgemäßen Verbindungen besteht in der Anfärbung von Latices, wobei vorzugsweise Verbindungen der Formel (Ia) und/oder (Ib) verwendet werden, in denen der Rest R⁷ möglichst lipophil ist. Da sich solche Latices sehr gut zur Verwendung in der Diagnostik eignen, ist weiterer Gegenstand der Erfindung die Verwendung solcher Latices in der Diagnostik.

Weiterer Gegenstand ist auch die Verwendung der erfindungsgemäßen Verbindungen als Absorptionsmedium für Sonnenkollektoren.

Weiterer Gegenstand der vorliegenden Erfindung ist ein Verfahren gemäß Patentanspruch 18 zur Bestimmung einer ersten immunologisch bindefähigen Substanz, das dadurch gekennzeichnet ist, daß ein Konjugat einer erfindungsgemäßen Verbindung mit einer zweiten immunologisch bindefähigen Substanz, die gleich oder verschieden mit der ersten Substanz sein kann, verwendet wird und die durch eine immunologische Bindungsreaktion, welche für die erste Substanz spezifisch ist, verursachte Absorption oder Fluoreszenzänderung der erfindungsgemäßen Verbindung als Maß für die Menge der in der Probe enthaltenen zu bestimmenden Substanz bestimmt.

Die nachstehenden Beispiele sollen die Erfindung näher erläutern, ohne sie darauf zu beschränken.

### BEISPIELE

### Beispiel 1

### 7-Methoxy-2,2,4-trimethyl-1,2,3,4-tetrahydrochinolin

30 g (0,147 Mol) 7-Methoxy-2,2,4-trimethyl-1,2-dihydrochinolin (hergestellt nach A. Rosowsky und E.J. Modest, J. org. Chem. 30 (1965) 1832) werden in 100 ml Methanol gelöst und mit 2,5 g Palladium an Aktivkohle (10 %) versetzt. Die Substanz wird 12 Stunden bei Raumtemperatur und einem Druck von 70 bar Wasserstoff hydriert. Nach Ablauf der Reaktion wird der katalysator abfiltriert und das Lösungsmittel am Rotationsverdampfer entfernt. Das Produkt kristallisiert in Form eines wachsartigen, hellgrünen Produktes aus. Ausbeute: 95 %

Dünnschichtchromatographie: Kieselgel / Chloroform + 2 % Ethanol

- NMR - Daten: in CDCl₃

| | | |
|---|---|---|
| δ = 1,2 ppm | 2 -CH₃ | Singulett |
| δ = 1,5 ppm | -CH₃ + -CH₂- | Multiplett |
| δ = 2,8 ppm | -C⁴-H | Multiplett |
| δ = 3,6 ppm | -NH- | Singulett |
| δ = 3,7 ppm | -OCH₃- | Singulett |
| δ = 6,1 ppm | C⁸-H | Dublett |
| δ = 6,3 ppm | C⁶-H | Quartett |
| δ = 7,1 ppm | C⁵-H | Dublett |
| δ = 7,24 ppm | CHCl₃ | |

### Beispiel 2

### N-Ethyl-7-methoxy-2,2,4-trimethyl-1,2-dihydrochinolin

10 g (0,049 Mol) 7-Methoxy-2,2,4-trimethyl-1,2-dihydrochinolin (hergestellt nach A. Rosowsky und E.J. Modest, l.c.) werden in 100 ml trockenem Acetonitril gelöst und mit 13,1 g (0,1 Mol) wasserfreiem Kaliumcarbonat und 9,3 g (0,06 Mol) Ethyljodid 18 Stunden am Rückfluß gekocht. Anschließend wird die abgekühlte Reaktionsmischung filtriert und das Lösungsmittel abdestilliert. Das Rohprodukt ist ein hellgrün gefärbtes Öl. Ausbeute: 80 %.

Dünnschichtchromatographie: Kieselgel / Methylenchlorid - NMR - Daten: in CDCl₃

| | | |
|---|---|---|
| δ = 1,3 ppm | 2 -CH₃ + -CH₃ (Ethyl-Gruppe) | Multiplett |
| δ = 1,95 ppm | Vinyl -CH₃ | Singulett |
| δ = 3,7 ppm | -OCH₃- | Singulett |
| δ = 4,3 ppm | -CH₂- (Ethyl-Gruppe) | Quartett |
| δ = 5,15 ppm | Vinyl -H | Singulett |
| δ = 6,2 ppm | C⁸-H + C⁶-H | Multiplett |
| δ = 6,95 ppm | C⁵-H | Dublett |
| δ = 7,24 ppm | CHCl₃ | |

### Beispiel 3

### N-Ethyl-7-hydroxy-2,2,4-trimethyl-1,2-dihydrochinolin

20 g (0,086 Mol) des nach Beispiel 2 hergestellten N-Ethyl-7-methoxy-2,2,4-trimethyl-1,2-dihydrochinolins werden mit 50 ml Eisessig und 50 ml Bromwasserstoffsäure (45 %) 6 Stunden zum Sieden erhitzt. Die abgekühlte Reaktionsmischung wird mit 200 ml Wasser und 300 ml Chloroform versetzt. Anschließend wird die Reaktionsmischung mit 30 %-iger wässriger Natronlauge unter Kühlung neutralisiert. Die Phasen werden getrennt und die wäßrige Phase noch zweimal mit je 100 ml Chloroform extrahiert. Die organischen Phasen werden vereinigt und über Calciumchlorid getrocknet. Nach vollständiger Entfernung des Lösungsmittels am Rotationsverdampfer bleibt ein hellgrünes Öl zurück. Ausbeute: 85 %.

### Beispiel 4

### N-Ethyl-7-methoxy-2,2,4-trimethyl-1,2,3,4-tetrahydrochinolin

Das nach Beispiel 1 erhaltene 7-Methoxy-2,2,4-trimethyl-1,2,3,4-tetrahydrochinolin wird analog der im Beispiel 2 angegebenen Alkylierungsmethode in das N-Ethyl-Derivat überführt. Ausbeute: 90 %.

### Beispiel 5

### N-Ethyl-7-hydroxy-2,2,4-trimethyl-1,2,3,4-tetrahydrochinolin

Das nach Beispiel 4 erhaltene N-Ethyl-7-methoxy-2,2,4-trimethyl-1,2,3,4-tetrahydrochinolin wird wie im Beispiel 3 beschrieben in die 7-Hydroxy-Verbindung überführt.

### Beispiel 6

### Herstellung der Verbindung (3)

4 g (0,018 mol) N-Ethyl-7-hydroxy-2,2,4-trimethyl-1,2-dihydrochinolin, 4,3 g (0,015 mol) Tetrachlorphthalsäureanhydrid und 2,5 g (0,018 mol) Zinkchlorid werden gründlich miteinander vermischt und 5 h im Ölbad auf 170°C erhitzt. Die abgekühlte Schmelze wird pulverisiert, mit 500 ml Ethanol ausgekocht und filtriert. Man versetzt das Filtrat zuerst mit 100 ml 60 %-iger Perchlorsäure und anschließend tropfenweise mit 400 ml Wasser. Der ausgefallene Farbstoff wird abfiltriert und getrocknet.

Säulenchromatographie:
Kieselgel / Chloroform + 4 % Ethanol
Die vereinigten Farbstoffreaktionen werden zur Trockene eingeengt, in 200 ml Ethanol aufgenommen, filtriert und tropfenweise mit 50 ml 60 %-iger Perchlorsäure versetzt.
Anschließend tropft man ca. 600 ml Wasser zu. Der abfiltrierte Farbstoff wird mit Ether gewaschen und getrocknet.

- NMR - Daten: in DMSO-d₆

| | | |
|---|---|---|
| δ = 1,3 ppm | -CH₃(Ethyl-Gruppe) | Triplett |
| δ = 1,5 ppm | -CH₃ | Dublett |
| δ = 1,85 ppm | Vinyl-CH₃ | Singulett |
| δ = 2,5 ppm | DMSO | |
| δ = 3,75 ppm | -CH₂- (Ethyl-Gruppe) | Quartett |
| δ = 5,75 ppm | Vinyl-H | Singulett |
| δ = 6,7 ppm | Aromaten-H | Singulett |
| δ = 6,9 ppm | Aromaten-H | Singulett |

### Beispiel 7

### Herstellung der Verbindung (4)

4 g (0,018 Mol) des nach Beispiel 5 erhaltenen N-Ethyl-7-hydroxy-2,2,4-trimethyl-1,2,3,4-tetrahydrochinolins, 4,3 g (0,015 Mol) Tetrachlorphthalsäureanhydrid und 2,5 g (0,018 Mol) Zinkchlorid werden gründlich miteinander vermischt und 5 Stunden im Ölbad auf 170°C erhitzt. Die abgekühlte Schmelze wird pulverisiert, mit 500 ml Ethanol ausgekocht und filtriert. Man versetzt das Filtrat zuerst mit 100 ml 60 %-iger Perchlorsäure, und anschließend tropfenweise mit 400 ml Wasser. Der ausgefallene Farbstoff wird abfiltriert und säulenchromatographisch gereinigt (Alox N/Ethanol).

### Beispiel 8

### Herstellung der Verbindung (6)

1 g (5,2 mmol) N-Ethyl-7-methoxy-1,2,3,4-tetrahydrochinolin werden in 15 ml trockenem Methylenchlorid gelöst und im Eisbad unter Stickstoffschutz mit 0,5 ml Trifluoressigsäure und 3,2 g (15 mmol) Trifluoressigsäureanhydrid versetzt. Die Reaktionsmischung wird anschließend 72 h bei Raumtemperatur unter Luftausschluß verrührt. Danach destilliert man das Methylenchlorid und überschüssiges Trifluoressigsäureanhydrid ab. Der ölige Rückstand wird mit 20 ml 70 %-iger Schwefelsäure versetzt und 30 min im Ölbad auf 130°C erhitzt. Man versetzt die abgekühlte rote Lösung unter Kühlung mit 300 ml Ethanol und 50 ml 60 %-iger Perchlorsäure. Dazu tropft man langsam 250 ml Wasser. Der ausgefallene Farbstoff wird abfiltriert, mit Ether gewaschen und getrocknet.

### Chromatographie: Kieselgel / Chloroform + 10 % Ethanol

- NMR - Daten: in d₆-DMSO

| | | |
|---|---|---|
| δ = 1,2 ppm | -CH₃ (Ethyl-Gruppe) | Triplett |
| δ = 1,95 ppm | -C³ʼ⁹ H₂ | Quintett |
| δ = 2,5 ppm | DMSO | |
| δ = 2,85 ppm | -C⁴ʼ³ H₂- | Triplett |
| δ = 3,7 ppm | -Cʼ¹⁰ H₂- + -CH₂- (Ethylgruppe) | |
| δ = 6,95 ppm | Aromaten-H (-C¹ʼ¹⁴ -H) | Singulett |
| δ = 7,65 ppm | Aromaten-H (-C⁵ʼ⁷ -H) | Singulett |

### Beispiel 9

### Herstellung von Verbindung (15)

Unter Einsatz der entsprechenden Ausgangsprodukte (N-Ethyl-7-methoxy-2,2,4-trimethyl-1,2-dihydrochinolin gemäß Beispiel 2; Trifluoressigsäureanhydrid) wird die Verbindung (15) analog dem im Beispiel 8 angegeben Verfahren synthetisiert. Zur Farbstoffbildung wird der ölige Rückstand in 70 %-iger Schwefelsäure 1 Stunde auf 140°C erhitzt. Die Reinigung erfolgt analog Beispiel 8.

### Beispiel 10

### Herstellung von Verbindung (16)

1,62 g (5 mMol) 3-(7-Methoxy-2,2,4-trimethyl-1,2-dihydrochinol-l-yl)-propansulfonsäure werden in 50 ml trockenem Methylenchlorid suspendiert und mit 4 ml (5,95 g = 0,028 Mol) Trifluoressigsäureanhydrid versetzt. Die sich dunkelgrün färbende Lösung wird unter Luftabschluß 12 Stunden bei Raumtemperatur verrührt; anschließend wird abfiltriert und das Lösungsmittel vollständig abdestilliert. Der Rückstand wird mit 20 ml 70 %-iger Schwefelsäure versetzt und 1 Stunde bei 130°C erhitzt. Die rote, abgekühlte Lösung wird bis zu einem pH-Wert von 4 bis 5 mi Kaliumhydrogencarbonat versetzt. Die Paste wird getrocknet und anschließend in einer Lösung aus Chloroform mit 10 % Methanol suspendiert. Die abfiltrierte Farbstofflösung wird chromatographisch gereinigt (Kieselgel/Chloroform (250 ml) + Ethanol (250 ml) + Perchlorsäure (60 %-ig; 0,5 ml)).

Die 3-(7-Methoxy-2,2,4-trimethyl-1,2-dihydrochinol-1-yl)-propansulfonsäure wurde wie folgt hergestellt:

10,16 g (0,05 mol) 7-Methoxy-2,2,4-trimethyl-1,2-dihydrochinolin werden mit 6,7 g (0,055 mol) Propansulfon unter Stickstoffschutz 2 h auf 150°C erhitzt. Die erkaltete, erstarrte Schmelze wird in 50 ml 10 %-iger wäßriger Natriumcarbonat-Lösung gelöst und filtriert. Die Lösung wird anschließend mit 20 %-iger Salzsäure angesäuert. Der ausfallende Niederschlag wird abfiltriert, mit Aceton gewaschen und getrocknet.
Ausbeute: 80 %
Farbloser Feststoff

Dünnschichtchromatographie: Kieselgel / Chloroform + 30 % Methanol

### Beispiel 11

### Herstellung von Verbindung (11)

1,49 g (4,4 mmol) 4-(7-Methoxy-2,2,4-trimethyl-1,2-dihydrochinol-1-yl)-butansulfonsäure und 0,99 g (4 mmol) 4-(7-Methoxy-1,2,3,4-tetrahydrochinol-1-yl)-buttersäure werden in 50 ml Methylenchlorid suspendiert und mit 4 g (0,019 mol) Trifluoressigsäureanhydrid versetzt. Die Lösung wird 12 h unter Luftausschluß verrührt, anschließend filtriert und zur Trockene einrotiert. Der Rückstand wird mit 25 ml 70 %-iger Schwefelsäure versetzt und 30 min auf 130°C erhitzt. Man versetzt die rote Lösung mit Kaliumhydrogencarbonat bis zu einem pH-Wert von 4 - 5. Die zähe Paste wird mehrmals in einer Lösung aus Chloroform mit 20 % Methanol suspendiert. Man filtriert und engt die Lösung zur Trockene ein. Der Rückstand wird chromatographisch gereinigt (Kieselgel). Man trennt zuerst mit einem Gemisch aus Chloroform mit 15 % Ethanol und 0,1 % Perchlorsäure (60 %-ig) Farbstoff (7) ab. Anschließend steigert man die Polarität des Fließmittels durch den Zusatz von 20 % Ethanol und erhält den Farbstoff (11).

### Beispiel 12

### Herstellung von Verbindung (21)

0.5 g (2.6 mmol) N-Ethyl-7-hydroxy-2,2,4-trimethyl-1,2-dihydrochinolin, 0.3 g (3 mmol) Bernsteinsäureanhydrid und 0.4 g (3 mmol) Zinkchlorid werden 2 h auf 180 °C erhitzt. Die abgekühlte Schmelze wird in Ethanol gelöst, filtriert und mit 10 ml 60 %-iger Perchlorsäure versetzt. Dazu tropft man langsam 1 l Wasser. Der Niederschlag wird abfiltriert und getrocknet.

Dünnschichtchromatographie: Kieselgel / Chloroform (85 ml) + Ethanol (15 ml) + Perchlorsäure (1 ml) (60 %)

Säulenchromatographie: Kieselgel / Chloroform + 5 % Ethanol

NMR-Daten: in d₆-DMSO

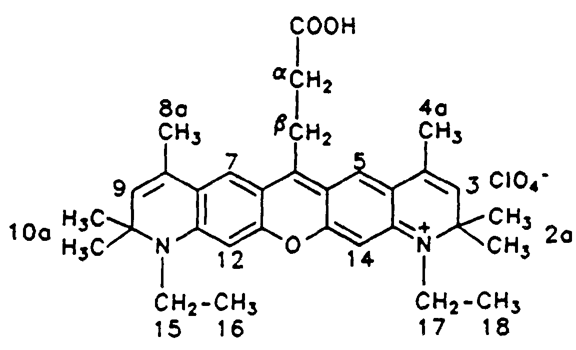

| Chemische Verschiebung δ in ppm | Signalform | Integration | Zuordnung |
|---|---|---|---|
| 1,1 | T | 6H | H-16, H-18 |
| 1,5 | S | 12H | H-2a, H-10a |
| 2,1 | S | 6H | H-4a, H-8a |
| 2,54 | - | - | DMSO |
| 2,75 | T | 2H | H-α |
| 3,7 | M | 6H | H-β, H-15, H-17 |
| 5,77 | S | 2H | H-3, H-9 |
| 6,75 | S | 2H | H-12, H-14 |
| 7,58 | S | 2H | H-5, H-7 |

### Beispiel 13

### Herstellung von Verbindung (22)

2 g (9,6 mmol) N-(2-Hydroxyethyl)-7-methoxy-1,2,3,4-tetrahydrochinolin werden in 15 ml Methylenchlorid gelöst und unter Stickstoffschutz mit 10 g (0,048 mol) Trifluoressigsäureanhydrid versetzt. Die Reaktionsmischung wird 12 h bei Raumtemperatur verrührt. Anschließend destilliert man das Methylenchlorid und überschüssiges Trifluoressigsäureanhydrid ab. Der ölige Rückstand wird mit 20 ml 70 %-iger Schwefelsäure versetzt und 30 min im Ölbad auf 130°C erhitzt. Nach der Kondensation versetzt man die abgekühlte schwefelsaure Lösung mit 100g Eis und 30 ml 60 %-iger Perchlorsäure. Die Lösung bleibt ca. 12 h in der Kälte stehen und wird anschließend filtriert. Eine Extraktion der Muterlauge erfolgt danach solange mit Chloroform, bis die wäßrige Phase nur noch schwach blau gefärbt ist. Die vereingten organischen Phasen werden über Na₂SO₄ getrocknet und eingeengt. Die verbleibende konzentrierte Farbstofflösung wird chromatographisch gereinigt.

Dünnschichtchromatographie: Kieselgel / Chloroform + 5 % Ethanol

Säulenchromatographie: Kieselgel / Chloroform + 5 % Ethanol + 1 % Trifluoressigsäure

-NMR-Daten: in CDCl₃

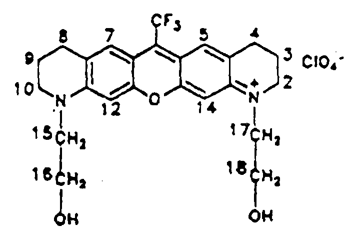

| Chemische Verschiebung δ in ppm | Signalform | Integration | Zuordnung |
|---|---|---|---|
| 2,0 | T | 4H | H-3, H-9 |
| 2,85 | T | 4H | H-4, H-8 |
| 3,7 | M | 8H | H-2, H-10 |
| | | | H-15, H-17 |
| 3,95 | T | 4H | H-16, H-18 |
| 6,9 | S | 2H | H-12, H-14 |
| 7,55 | S | 2H | H-5, H-7 |

### Beispiel 14

### Herstellung von Verbindung (23)

1,82 g (0,011 mol) 7-Methoxy-1,2,3,4-tetrahydrochinolin werden mit 2,04 g (0,011 mol) 1-Bromo-2-(2-methoxyethoxy-) ethan und 1,42 g (0,11 mol) Hünig-Base 12 h bei 120°C verrührt. Nach dem Abkühlen wird die Lösung filtriert, das abfiltrierte Salz mit wenig Aceton gewaschen und die Mutterlauge zur Trockene eingeengt. Der Rückstand wird mit 20 ml Methylenchlorid und 0,5 ml Trifluoressigsäure versetzt. Anschließend kühlt man die Lösung im Eis/NaCl-Bad ab und tropft 5 g Trifluoressigsäureanhydrid zu. Die Lösung wird 12 h verrührt, anschließend zur Trockene eingeengt und mit 20 ml 70 %-iger Schwefelsäure versetzt. Die Lösung erhitzt man danach 30 min auf 130°C. Die Aufarbeitung der abgekühlten Lösung erfolgt analog zu Farbstoff JA 49.

Chromatographie (Edukt): Kieselgel / Methylenchlorid + 5 % Ethanol

### Beispiel 15

### Herstellung von Verbindung (24)

- Präparation: analog zu Farbstoff 23 unter Verwendung von 2-2[2-(2-Chloro-ethoxy)-ethoxy]-ethanol
- Chromatographie (Edukt): Kieselgel / Chloroform + 5 % Ethanol + 0,2 % Trifluoressigsäure

### Beispiel 16

### Herstellung von N-Hydroxysuccinimidestern

100 mg eines entsprechenden Carbonsäure-Derivats (vorzugsweise eines Säurechlorids) werden unter den für die Herstellung von N-Hydroxysuccinimidestern üblichen Bedingungen mit Dicyclohexylcarbodiimid und N-Hydroxysuccinimid umgesetzt (Lösungsmittel: Tetrahydrofuran oder Dimethylformamid; Raumtemperatur; 4 Stunden). Nach Entfernung des Lösungsmittels erfolgt eine Reinigung durch Flash-Chromatographie.

### Beispiel 17

### Kupplung aktivierter erfindungsgemäßer Farbstoffe an Proteine (z.B. eines nach Beispiel 11 hergestellten Farbstoffes)

10 mg Protein werden in 1 ml NaHCO₃-Puffer, 0,1 Mol/l, pH = 8,5, gelöst. Dazu gibt man eine Lösung des Farbstoffes in DMSO (je nach gewünschter Anzahl der gekoppelten Farbstoffmoleküle wird mit einem entsprechenden Überschuß gearbeitet). Die Reaktionslösung wird 1 Stunde bei Raumtemperatur geschüttelt. Das Konjugat wird über eine G 50 Sephadex-Säule (Laufmittel NaHCO₃-Puffer) vom freien Farbstoff abgetrennt, ausreichend gegen H₂O dialysiert und lyophilisiert.

### Beispiel 18

### Beladung von Latex-Partikeln mit lipophilen erfindungsgemäßen Farbstoffen

5 ml 1 %-ige Latexsuspension (z.B. Polystyrollatices der Firma Polyscience) werden mit 1 mg des erfindungsgemäßen Farbstoffes, gelöst in 200 µl Chloroform, versetzt und ca. 20 Stunden geschüttelt. Das Lösungsmittel wird durch Einleiten von Stickstoff entfernt, und die Abtrennung der freien Farbstoffpartikel erfolgt durch Zentrifugieren bzw. Filtration.

### Beispiel 19

### Bestimmung der Fluoreszenz-Quantenausbeute von Verbindung (15)

Es werden Lösungen des erfindungsgemäßen Farbstoffes und von Rhodamin 800 (vgl. R. Raue, H. Harnisch, K.H. Drexhage, Heterocycles 21(1), 167, (1984)) (Referenzsubstanz) in Ethanol hergestellt und die Konzentration so eingestellt, daß die Extinktion jeder Lösung bei der Wellenlänge 676 nm den Wert 1,7 mm hat. Dann werden die Fluoreszenz-Spektren der Lösung in einer 1 mm Küvette (Front Surface Messung) mit dem Spektralfluorimeter Fluorolog 2 der Firma Spec (Anregungswellenlänge 676 nm) unter Verwendung derselben Geräteparameter gemessen und die Flächen unter den Fluoreszenz-Kurven bestimmt. Durch Vergleich mit dem Referenz-Farbstoff Rhodamin 80, dessen Quantenausbeute 21 % beträgt (vgl. R. Sens, Dissertation, Siegen 1984) ergibt sich z.B. für die erfindungsgemäße Verbindung (15) eine Quantenausbeute von 55 %.

## Patentansprüche

1. Pentacyclische Derivate der allgemeinen Formeln (Ia), (Ib) und (Ic) 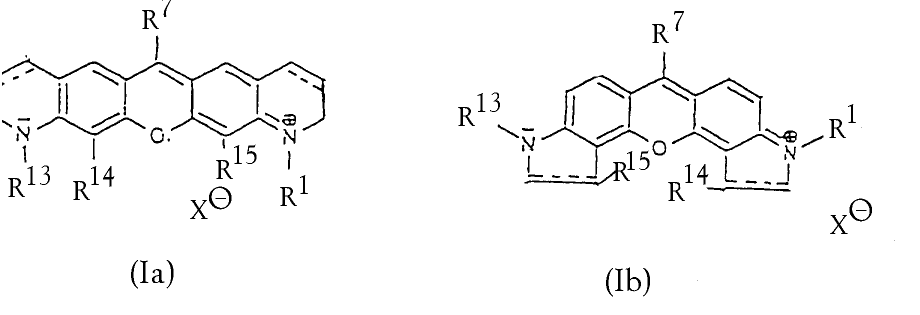 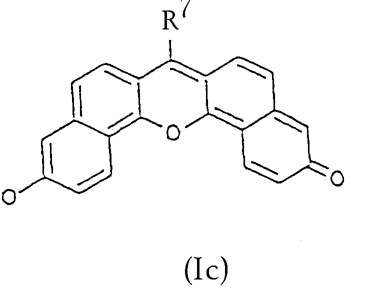 worin R¹ und R¹³ gleich oder verschieden sind und bedeuten: Wasserstoff, Alkyl mit 1 bis 20 Kohlenstoffatomen, Polyoxyhydrocarbyl-Einheiten, Phenyl, Phenylalkyl mit 1 bis 3 Kohlenstoffatomen in der Alkylkette, worin die Alkyl- oder/und Phenylreste durch eine oder mehrere Hydroxy-, Halogen-, Sulfo-, Carboxy- oder Alkoxycarbonylgruppen, worin Alkoxy 1 bis 4 Kohlenstoffatome besitzen kann, substituiert sein können;
R⁷ eine durch mindestens ein Halogen substituierte Alkylgruppe mit 1 bis 20, vorzugsweise 1 bis 7 Kohlenstoffatomen oder eine Phenylgruppe, die durch eine in o-Stellung zu dem an das pentacyclische Ringsystem gebundene Kohlenstoffatom befindliche Carboxy- oder Alkoxycarbonylgruppe und mindestens 1 Halogen substituiert ist, und worin Alkoxy 1 bis 4 Kohlenstoffatome besitzen kann, oder eine Carboxyalkylgruppe oder eine Carboxymethylenoxy-alkyloxygruppe bedeutet;
die beiden strichliniert eingezeichneten Bindungen bedeuten, daß die beiden durch die strichlinierte Bindung verbundenen Kohlenstoffatome durch eine Einfach- oder Doppelbindung miteinander verbunden sein können; wobei R¹⁴, R¹⁵ und die übrigen, nicht mit spezifischen Symbolen bezeichneten Positionen des pentacyclischen Grundkörpers durch Alkylgruppen mit 1 bis 20 Kohlenstoffatomen substituiert sein können; wobei R¹ bzw. R¹³ bei Verbindungen der Formel Ia nicht mit Substituenten an den Positionen R¹⁵ bzw. R¹⁴ verbrückt sind, und X⁻ ein Gegenion ist; sowie deren zumindest an einem der Reste R¹, R⁷ oder/und R¹³ aktivierten Derivate.

2. Verbindungen nach Anspruch 1,
**dadurch gekennzeichnet,**
daß R¹ oder/und R¹³ Wasserstoff, Alkyl mit 1 bis 4 Kohlenstoffatomen, das durch eine Hydroxy-, Halogen-, Carboxy-, Alkoxycarbonyl- oder Sulfogruppe substituiert sein kann, oder Benzyl bedeuten.

3. Verbindungen nach Anspruch 1,
**dadurch gekennzeichnet,**
daß R¹ oder/und R¹³ Polyoxyhydrocarbyl-Einheiten sind.

4. Verbindungen nach Anspruch 3,
**dadurch gekennzeichnet,**
daß die Polyoxyhydrocarbyl-Einheiten Polyether, Polyole, lösliche Carbohydrate und Derivate davon oder wasserlösliche Polymere sind.

5. Verbindungen nach einem der Ansprüche 1 bis 4,
**dadurch gekennzeichnet,**
daß R⁷ eine perhalogenierte, insbesondere perfluorierte Alkylgruppe mit 1 bis 3 Kohlenstoffatomen ist.

6. Verbindungen nach einem der Ansprüche 1 bis 4,
**dadurch gekennzeichnet,**
daß R⁷ eine Carboxyalkylgruppe mit einer Alkylkettenlänge von O bis 10 oder eine Carboxylmethylen-oxy-alkyloxygruppe ist.

7. Verbindungen nach einem der Ansprüche 1 bis 6,
**dadurch gekennzeichnet,**
daß eine oder beide der strichlinierten Bindungen der Formeln Ia oder/und Ib Doppelbindungen sind.

8. Verbindungen nach einem der Ansprüche 1 bis 7,
**dadurch gekennzeichnet,**
daß das Gegenion ein Perchlorat- oder Heptafluorbutyrat-Ion oder ein aus einem der Reste gebildetes Carboxy- oder Sulfo-Ion ist.

9. Verbindungen nach einem der Ansprüche 1 bis 8,
**dadurch gekennzeichnet,**
daß das von einem der Reste R¹, R⁷ oder/und R¹³ abgeleitete aktivierte Derivat ein von der Carboxylgruppe abgeleiteter N-Hydroxy-Succinimidester oder/und ein Säurechlorid ist.

10. Konjugate, die eine Verbindung nach einem der Ansprüche 1 bis 9 enthalten.

11. Verwendung einer Verbindung nach einem der Ansprüche 1 bis 9 als Absorptions- oder/und Fluoreszenzfarbstoff.

12. Verwendung einer Verbindung nach einem der Ansprüche 1 bis 9 als Anregungs- und Emissionsmedium in einem Farbstofflaser.

13. Verwendung einer Verbindung nach einem der Ansprüche 1 bis 9 oder eines Konjugats nach Anspruch 10 in diagnostischen Systemen.

14. Verwendung einer Verbindung nach einem der Ansprüche 1 bis 9 als Marker für Hapten/Antikörper/Protein-Konjugate.

15. Verwendung der Konjugate nach Anspruch 14 in diagnostischen Systemen.

16. Verwendung nach Anspruch 14 oder 15,
**dadurch gekennzeichnet,**
daß man eine Verbindung der Formel (Ia) oder/und (Ib) verwendet, in der die Reste R¹ und R¹³ so ausgewählt sind, daß sie möglichst hydrophil sind.

17. Verwendung nach Anspruch 14 oder 15,
**dadurch gekennzeichnet,**
daß die Kupplung an das Konjugat über die Reste R¹, R⁷ oder/und R¹³ erfolgt.

18. Verwendung nach Anspruch 17,
**dadurch gekennzeichnet,**
daß die Kupplung mittels einer N-Hydroxy-Succinimidgruppierung erfolgt.

19. Verwendung einer Verbindung nach einem der Ansprüche 1 bis 9 zur Anfärbung von Latices.

20. Verwendung nach Anspruch 19,
**dadurch gekennzeichnet,**
daß man eine Verbindung der Formel (Ia) oder/und (Ib) verwendet, worin der Rest R⁷ möglichst lipophil ist.

21. Verwendung nach Anspruch 19 oder 20,
**dadurch gekennzeichnet,**
daß die Latices in der Diagnostik verwendet werden.

22. Verwendung einer Verbindung nach einem der Ansprüche 1 bis 9 als Absorptionsmedium für Sonnenkollektoren.

23. Verfahren zur Bestimmung einer ersten immunologisch bindefähigen Substanz,
**dadurch gekennzeichnet,**
daß ein Konjugat aus einer Verbindung nach einem der Ansprüche 1 bis 9 mit einer zweiten immunologisch bindefähigen Substanz, die gleich oder verschieden mit der ersten Substanz sein kann, verwendet wird und daß die durch eine immunologische Bindungsreaktion, welche für die erste Substanz spezifisch ist, verursachte Absorptions- oder/und Fluoreszenzänderung der Verbindung nach einem der Ansprüche 1 bis 9 als Maß für die Menge der in der Probe enthaltenen zu bestimmenden Substanz bestimmt wird.

## Claims

1. Pentacyclic derivatives having the general formulae (Ia), (Ib) and (Ic) 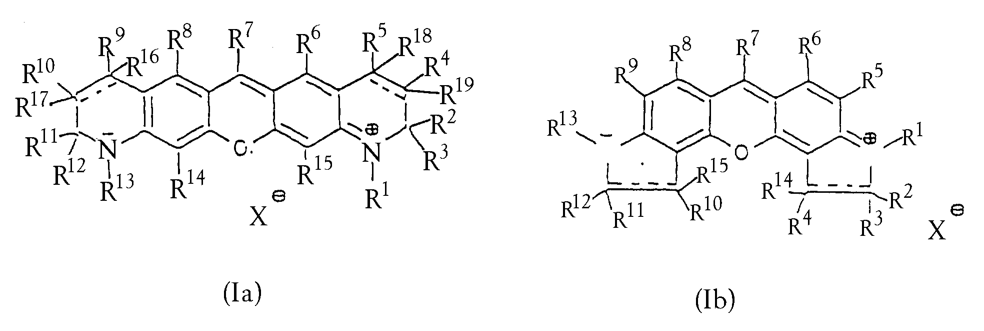 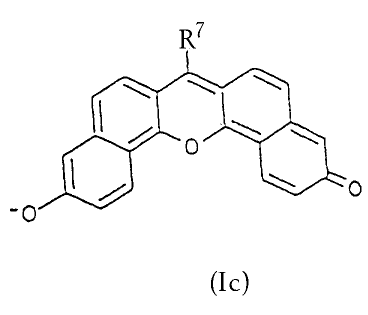 in which R¹ and R¹³ are the same or different and denote: hydrogen, alkyl with 1 to 20 carbon atoms polyoxyhydrocarbyl units, phenyl, phenylalkyl with 1 to 3 carbon atoms in the alkyl chain, wherein the alkyl residues or/and phenyl residues can be substituted by one or several hydroxy, halogen, sulfo, carboxy or alkoxycarbonyl groups in which alkoxy can have 1 to 4 carbon atoms;
R⁷ denotes an alkyl group with 1 to 20, preferably 1 to 7 carbon atoms, substituted by at least one halogen, or denotes a phenyl group which is substituted by a carboxy or alkoxycarbonyl group located at the o-position relative to the carbon atom bound to the pentacyclic ring system and by at least one halogen, wherein alkoxy can have 1 to 4 carbon atoms, or a carboxyalkyl group or a carboxymethylene-oxy-alkyloxy group;
the two bonds marked by a broken line mean that the two carbon atoms joined by the bond marked by a broken line can be linked together by a single or double bond;
in which R¹⁴ and R¹⁵ and the other positions in the pentacyclic backbone not marked with specific symbols can be substituted by alkyl groups with 1 to 20 carbon atoms;
wherein R¹ and R¹³ in the case of compounds of formula Ia are not bridged with substituents at positions R¹⁵ or R¹⁴, and X is a counterion; as well as their derivatives activated at least at one of the residues R¹, R⁷ or/and R¹³.

2. Compounds as claimed in claim 1,
**wherein**
R¹ or/and R¹³ denote hydrogen, alkyl with 1 to 4 carbon atoms which can be substituted by a hydroxy, halogen, carboxy, alkoxycarbonyl or sulfo group, or benzyl.

3. Compounds as claimed in claim 1,
**wherein**
R¹ or/and R¹³ are polyoxyhydrocarbyl units.

4. Compounds as claimed in claim 3,
**wherein**
the polyoxyhydrocarbyl units are polyethers, polyols, soluble carbohydrates and derivatives thereof or water-soluble polymers.

5. Compounds as claimed in one of the claims 1 to 4,
**wherein**
R⁷ is a perhalogenated, especially perfluorinated, alkyl group with 1 to 3 carbon atoms.

6. Compounds as claimed in one of the claims 1 to 4,
**wherein**
R⁷ is a carboxyalkyl group with an alkyl chain length of 0 to 10 or a carboxylmethylene-oxyalkyloxy group.

7. Compounds as claimed in one of the claims 1 to 6,
**wherein**
one or both of the bonds of formulae Ia or/and Ib marked by a broken line are double bonds.

8. Compounds as claimed in one of the claims 1 to 7,
**wherein**
the counterion is a perchlorate or heptafluorobutyrate ion or a carboxy or sulfo ion formed from one of the residues.

9. Compounds as claimed in one of the claims 1 to 8,
**wherein**
the activated derivative derived from one of the residues R¹, R⁷ or/and R¹³ is a N-hydroxysuccinimide ester or/and an acid chloride derived from the carboxyl group.

10. Conjugates which contain a compound as claimed in one of the claims 1 to 9.

11. Use of a compound as claimed in one of the claims 1 to 9 as an absorption or/and fluorescent dye.

12. Use of a compound as claimed in one of the claims 1 to 9 as an excitation and emission medium in a dye laser.

13. Use of a compound as claimed in one of the claims 1 to 9 or of a conjugate as claimed in claim 10 in diagnostic systems.

14. Use as claimed in one of the claims 1 to 9 as a marker for hapten/antibody/protein conjugates.

15. Use of the conjugates as claimed in claim 14 in diagnostic systems.

16. Use as claimed in claim 14 or 15,
**wherein**
a compound of formula (Ia) or/and (Ib) is used in which the residues R¹ and R¹³ are selected so that they are as hydrophilic as possible.

17. Use as claimed in claim 14 or 15,
**wherein**
the coupling to the conjugate is carried out via the residues R¹, R⁷ or/and R¹³.

18. Use as claimed in claim 17,
**wherein**
the coupling is achieved by means of a N-hydroxysuccinimide group.

19. Use of a compound as claimed in one of the claims 1 to 10 for dyeing latices.

20. Use as claimed in claim 19,
**wherein**
a compound of formula (Ia) or/and (Ib) is used in which residue R⁷ is as lipophilic as possible.

21. Use as claimed in one of the claims 19 or 20,
**wherein**
the latices are used in diagnostics.

22. Use of a compound as claimed in one of the claims 1 to 9 as an absorption medium for solar collectors.

23. Method for the determination of a first immunologically bindable substance,
**wherein**
a conjugate of a compound as claimed in one of the claims 1 to 9 with a second immunologically bindable substance which can be the same as or different from the first substance is used and the change in absorbance or/and fluorescence of the compound as claimed in one of the claims 1 to 9 caused by an immunological binding reaction which is specific for the first substance is determined as a measure for the amount of the substance to be determined present in the sample.

## Revendications

1. Dérivés pentacycliques selon les formules générales (Ia), (Ib) et (Ic) 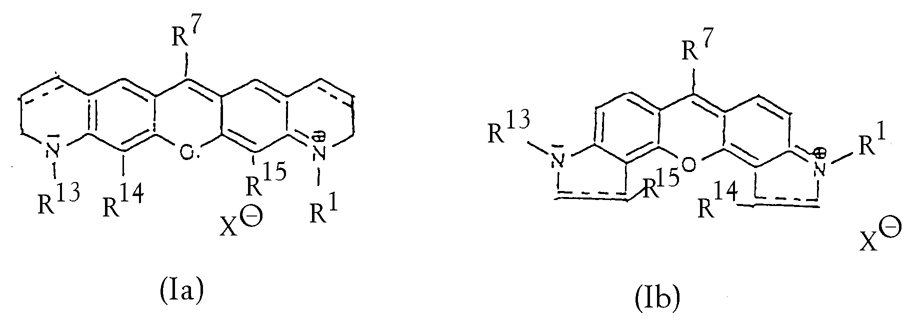 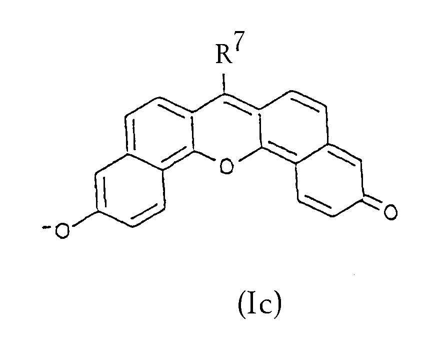 où R¹ et R¹³ sont identiques ou différents et représentent: l'hydrogène, un reste alkyle de 1 à 20 atomes de carbone, des unités polyoxyhydrocarbyle, un reste phényle, phénylalkyle ayant 1 à 3 atomes de carbone dans la chaîne alkyle, où les restes alkyle et/ou phényle peuvent être substitués par un ou plusieurs groupes hydroxyle, halogène, sulfo, carboxyle ou alcoxy, alcoxy pouvant posséder 1 à 4 atomes de carbone;
R⁷ représente un groupe alkyle de 1 à 20, de préférence de 1 à 7 atomes de carbone, substitué par au moins un halogène, ou un groupe phényle qui est substitué par un groupe carboxyle ou alcoxy situé en position o par rapport à l'atome de carbone lié au système pentacyclique et par au moins un halogène, et où alcoxy peut posséder 1 à 4 atomes de carbone, ou un groupe carboxyalkyle ou un groupe carboxyméthylène-oxy-alkyloxy;
les deux liaisons représentées en pointillés signifient que les deux atomes de carbone liés par la liaison en pointillés peuvent être liés l'un à l'autre par une liaison simple ou une double liaison;
R¹⁴, R¹⁵ et les autres positions du corps fondamental pentacyclique qui ne sont pas désignées par des symboles spécifiques pouvant être substitués par des groupes alkyle de 1 à 20 atomes de carbone;
R¹ et R¹³, respectivement, dans le cas des composés de formule Ia, n'étant pas liés par pontage à des substituants aux positions R¹⁵ et R¹⁴, respectivement, et X étant un contre-ion, ainsi que leurs dérivés activés au moins au niveau de l'un des restes R¹, R⁷ et/ou R¹³.

2. Composés selon la revendication 1, caractérisés en ce que R¹ et/ou R¹³ représentent l'hydrogène, alkyle de 1 à 4 atomes de carbone qui peut être substitué par un groupe hydroxyle, halogène, carboxyle, alcoxycarbonyle ou sulfo, ou benzyle.

3. Composés selon la revendication 1, caractérisés en ce que R¹ et/ou R¹³ sont des unités polyoxyhydrocarbyle.

4. Composés selon la revendication 3, caractérisés en ce que les unités polyoxyhydrocarbyle sont des polyéthers, des polyols, des glucides solubles et des dérivés de ceux-ci ou des polymères hydrosolubles.

5. Composés selon l'une des revendications 1 à 4, caractérisés en ce que R⁷ est un groupe alkyle perhalogéné, en particulier perfluoré, de 1 à 3 atomes de carbone.

6. Composés selon l'une des revendications 1 à 4, caractérisés en ce que R⁷ est un groupe carboxyalkyle ayant une longueur de chaîne alkyle de 0 à 10 ou un groupe carboxyméthylène-oxy-alkyloxy.

7. Composés selon l'une des revendications 1 à 6, caractérisés en ce qu'une liaison en pointillés ou les deux liaisons en pointillés des formules Ia et/ou Ib sont des doubles liaisons.

8. Composés selon l'une des revendications 1 à 7, caractérisés en ce que le contre-ion est un ion perchlorate ou heptafluorobutyrate ou un ion carboxyle ou sulfo formé à partir de l'un des restes.

9. Composés selon l'une des revendications 1 à 8, caractérisés en ce que le dérivé activé dérivé de l'un des restes R¹, R⁷ et/ou R¹³ est un ester de N-hydroxysuccinimide et/ou un chlorure d'acide dérivé du groupe carboxyle.

10. Conjugués qui contiennent un composé selon l'une des revendications 1 à 9.

11. Utilisation d'un composé selon l'une des revendications 1 à 9 comme colorant par absorption et/ou fluorescence.

12. Utilisation d'un composé selon l'une des revendications 1 à 9 comme milieu d'excitation ou d'émission dans un laser à colorant.

13. Utilisation d'un composé selon l'une des revendications 1 à 9 ou d'un conjugué selon la revendication 10 dans des systèmes diagnostiques.

14. Utilisation d'un composé selon l'une des revendications 1 à 9 comme marqueur pour conjugués haptène/anticorps/protéine.

15. Utilisation des conjugués selon la revendication 14 dans des systèmes diagnostiques.

16. Utilisation selon la revendication 14 ou 15, caractérisée en ce que l'on utilise un composé de formule (Ia) et/ou (Ib) dans lequel les restes R¹ et R¹³ sont choisis de manière qu'ils soient le plus hydrophiles possible.

17. Utilisation selon la revendication 14 ou 15, caractérisée en ce que le couplage avec le conjugué a lieu par l'intermédiaire des restes R¹, R⁷ et/ou R¹³.

18. Utilisation selon la revendication 17, caractérisée en ce que le couplage a lieu au moyen d'un groupement N-hydroxysuccinimide.

19. Utilisation d'un composé selon l'une des revendications 1 à 9 pour la teinture de latex.

20. Utilisation selon la revendication 19, caractérisée en ce que l'on utilise un composé de formule (Ia) et/ou (Ib) dans lequel le reste R⁷ est le plus lipophile possible.

21. Utilisation selon la revendication 19 ou 20, caractérisée en ce que les latex sont utilisés en diagnostic.

22. Utilisation d'un composé selon l'une des revendications 1 à 9 comme milieu d'absorption pour collecteurs solaires.

23. Procédé pour déterminer une première substance capable de se lier de manière immunologique, caractérisé en ce qu'un conjugué d'un composé selon l'une des revendications 1 à 9 et d'une seconde substance capable de se lier de manière immunologique, qui peut être identique à la première substance ou être différente de celle-ci, est utilisé et en ce que la variation d'absorption et/ou de fluorescence du composé selon l'une des revendications 1 à 9 qui est provoquée par une réaction de liaison immunologique qui est spécifique pour la première substance est déterminée comme mesure de la quantité de substance à déterminer contenue dans l'échantillon.
